# EUROPEAN PATENT SPECIFICATION

(11) **EP 1 882 682 B1**
(45) Date of publication and mention of the grant of the patent: **08.07.2015**
(21) Application number: 06746221.8
(22) Date of filing: 10.05.2006
(51) Int. Cl.: C07C 253/34, C07C 255/03

(54) **HIGH-PURITY ACETONITRILE AND PROCESS FOR PRODUCING THE SAME**
HOCHREINES ACETONITRIL UND HERSTELLUNGSVERFAHREN DAFÜR
ACETONITRILE EXTREMEMENT PUR ET SON PROCEDE DE PRODUCTION

(30) Priority: 10.05.2005 JP 2005136820
(43) Date of publication of application: 30.01.2008
(73) Proprietor: Asahi Kasei Chemicals Corporation, Tokyo 101-8101 (JP)
(72) Inventor: SANO, Kazuhiko, Tokyo 100-8440 (JP); OHYAMA, Kikuo, Tokyo 100-8440 (JP); HINAGO, Hidenori, Tokyo 100-8440 (JP)
(74) Representative: Strehl Schübel-Hopf & Partner
(86) International application number: PCT/JP2006/309407
(87) International publication number: WO 2006/121081

(56) References cited:
- EP-A1- 0 890 572
- JP-A- 05 025 112
- JP-A- 11 035 542
- JP-A- 55 153 757

## Description

### [Technical Field]

The present invention relates to a process for producing highly pure acetonitrile.

### [Background Art]

At present, acetonitrile generally commercially available is mostly prepared by recovering and purifying crude acetonitrile obtained as a by-product in the production of acrylonitrile or methacrylonitrile by catalytic ammoxydation of propylene or isobutene with ammonia and molecular oxygen.

The crude acetonitrile obtained as a by-product contains impurities such as allyl alcohol, oxazole, water, acetone, hydrocyanic acid, acrylonitrile, methacrylonitrile, acrolein, propionitrile, cis- and trans-chrotononitrile, acrylic acid, methyl acrylate, methacrylic acid, methyl methacrylate, acetic acid, acrolein, methacrolein, and acetone, impurities impossible to analyze, and the like.

Acetonitrile is used as a solvent for chemical reactions, particularly a solvent for synthesis and purification of pharmaceutical intermediates, a mobile phase solvent for high-performance liquid chromatography, and the like. Also, recently, it is used as a solvent for DNA synthesis and purification, a solvent for synthesis of organic EL materials, a cleaning solvent for electronic parts, and the like. In view of such applications, its high purification is required. When one product can be used in various applications, such acetonitrile may be considered as more preferred one.

As a method of confirming the high purification, analysis of the impurities by an absorbance method in an ultraviolet region is very effective for analysis of a minute amount of impurities since the absorbance method in an ultraviolet region is an analytical method exhibiting a very high sensitivity.

Particularly, in the case that acetonitrile is used as a mobile phase solvent for high-performance liquid chromatography, an important item of quality is an absorbance in ultraviolet absorption at a wavelength of 200 nm. Recently, since the analysis can be performed at a higher sensitivity at a lower wavelength side, quality with a low absorbance in ultraviolet absorption at a wavelength of 200 nm is required. Even when the acetonitrile is high purity acetonitrile, in the case that a very minute amount of a compound having an absorbance in an ultraviolet region is present as an impurity, the acetonitrile is not satisfactory as a mobile phase solvent for high-performance liquid chromatography owing to its high absorbance.

On the other hand, no response may be observed against impurities having no absorption in an ultraviolet region and even when it is regarded as a high purity one based on the absorption analysis in an ultraviolet region, it is not assured that the other impurities are not contained. Propionitrile hardly has ultraviolet absorption and also is a compound most difficult to remove until a high level of removal among known impurities.

In applications to be used as the solvent or cleaning agent for the above applications, a small content of propionitrile is required.

There have been proposed various processes for purifying acetonitrile by removing impurities to a high extent.

For example, the present applicants have proposed a process for producing highly pure acetonitrile having an absorbance in ultraviolet absorption at a wavelength of 200 nm of 0.05 abs/cm or less by reacting purified acetonitrile with nascent oxygen and subsequently bringing the resulting product into contact with a substance selected from solid bases and adsorbents. Specifically, ozone is disclosed as the nascent oxygen and an anion-exchange resin as the adsorbent. A step of subjecting the resulting acetonitrile to further distillation treatment to produce highly pure acetonitrile has been also disclosed (see, e.g., Patent Document 1).

In addition, as similar processes, there have been disclosed processes for producing highly pure acetonitrile by steps of ozone treatment and column treatment with molecular sieves, active alumina, zirconia, charcoal, or graphite carbon (see, e.g., Patent Documents 2 and 3).

Although the acetonitrile obtained by such steps is excellent highly pure acetonitrile having a high purity and a very small absorbance in an ultraviolet region, it still suffers from problems that propionitrile is not sufficiently reduced, the steps are complex since it is produced via plurality of treating steps, and the cost largely increases since energy consumption for use in the purification increases.

In addition, there has been disclosed a process for producing highly pure acetonitrile wherein purified acetonitrile is obtained by distilling and purifying acetonitrile with a large load on distillation, i.e., a large reflux ratio using three distillation towers of a pressure distillation tower, a reduced-pressure distillation tower, and a pressure distillation tower and then highly pure acetonitrile is produced by passing the purified acetonitrile through a cation-exchange resin (see, e.g., Patent Document 4). This process suffers from problems that energy for pressure-reduction and pressurization is consumed and also a pressurization-resistant facility is necessary. Moreover, the process is not satisfactory for removal of impurities such as propionitrile.

In the process for producing acetonitrile with a small consumption of energy and using a simple facility, it is desired to produce highly pure acetonitrile having a small absorbance in an ultraviolet region, which has a small content of propionitrile for applications such as a solvent for synthesis and purification of pharmaceutical intermediates, a solvent for synthesis and purification of DNA, a solvent for synthesis of organic EL materials, and a cleaning solvent for electronic parts, and which is also purified to a level sufficiently usable as a mobile phase solvent for high-performance liquid chromatography.

In the conventional purification methods, it is difficult to achieve the quality satisfying such requests.

[Patent Document 1] JP-A-6-329610 (US Patent No. 5,629,443)
[Patent Document 2] US Patent No. 5,292,919
[Patent Document 3] US Patent No. 5,426,208
[Patent Document 4] JP-A-11-35542 (US Patent No. 2004/0176631)
[Patent Document 5] JP-A-58-124751
[Patent Document 6] JP-B-45-36490
[Patent Document 7] JP-A-55-153757
[Patent Document 8] JP-B-63-31458

JP 05 025 112 A discloses a method of obtaining pure acetonitrile from crude acetonitrile using a catalyst and an ozone-containing gas.

EP 0 890 572 A1 discloses a method of producing highly pure acetonitrile from crude acetonitrile containing water by triple distillation and subsequent contacting with an acidic ion exchange resin.

### [Disclosure of the Invention]

### [Problems to be Solved by the Invention]

An object of the invention is to provide a process for producing highly pure acetonitrile having a small absorbance in an ultraviolet region, which has a small content of propionitrile for applications such as a solvent for synthesis an purification of pharmaceutical intermediates, a solvent for synthesis and purification of DNA, a solvent for synthesis of organic EL materials, and a cleaning solvent for electronic parts, and which is also purified to a level sufficiently usable as a mobile phase solvent for high-performance liquid chromatography with suppressing an increase in cost to a minimum level by providing a process which requires a small consumption of energy and simple purification facility and steps.

### [Means for Solving the Problems]

As a result of extensive studies for solving the above problems, the present inventors have found that highly pure acetonitrile having a remarkably low content of propionitrile contained in the acetonitrile and a small absorbance in an ultraviolet absorption at a wavelength of 200nm is surprisingly obtained with suppressing an increase in cost to a minimum level by providing a process which requires a small consumption of energy for use in purification and simple purification facility and steps without containing any high-pressure step, which comprises mixing hydrous crude acetonitrile with an alkali, separating the resulting mixture into an acetonitrile phase and an aqueous phase, removing the aqueous phase, passing the resulting acetonitrile phase through a distillation step to obtain purified acetonitrile, and passing the resulting purified acetonitrile though a cation-exchange resin. They have also found that, thereby, it becomes possible to provide highly pure acetonitrile, which has a small content of propionitrile for applications such as a solvent for synthesis an purification of pharmaceutical intermediates, a solvent for synthesis and purification of DNA, a solvent for synthesis of organic EL materials, and a cleaning solvent for electronic parts, and which is also purified to a level sufficiently usable as a mobile phase solvent for high-performance liquid chromatography.

Namely, the invention relates to:
(1) A process for producing highly pure acetonitrile, which comprises mixing hydrous crude acetonitrile with an alkali, separating the resulting mixture into an acetonitrile phase and an aqueous phase, removing the aqueous phase to remove the moisture contained in the acetonitrile to 10% by weight or less, distilling the resulting acetonitrile phase from 0.05 MPa to 0.27 MPa of pressure of the distillation tower to obtain purified acetonitrile, and passing the resulting purified acetonitrile through a cation-exchange resin to obtain highly pure acetonitrile,
   wherein the highly pure acetonitrile is highly pure acetonitrile having a content of propionitrile of 150 ppm by weight or less and an absorbance in ultraviolet absorption at 200 nm of 0.3 abs/cm or less;
(2) The process for producing highly pure acetonitrile according to (1), wherein the distillation step comprises a step of refluxing from a top of a distillation tower to the distillation tower via a condenser and discharging the remainder to outside of the distillation tower and a step of recovering purified acetonitrile from a bottom of the tower, and a ratio of the reflux of the distillation tower is from 5 to 50;
(3) The process for producing highly pure acetonitrile according to (1) or (2), wherein the alkali is sodium hydroxide and/or potassium hydroxide;
(4) The process for producing highly pure acetonitrile according to any one of (1) to (3), wherein the acetonitrile phase is distilled to carry out removal of components having a boiling point lower than that of acetonitrile and removal of components having a boiling point higher than that of acetonitrile;
(5) The process for producing highly pure acetonitrile according to any one of (1) to (4), wherein the cation-exchange resin is a strongly acidic cation-exchange resin.

### [Advantages of the Invention]

Since the process of the invention employs a process which requires a small consumption of energy and simple purification facility and steps, it becomes possible to provide highly pure acetonitrile having a low absorbance in ultraviolet absorption at a wavelength of 200 nm, which has a small content of propionitrile for applications such as a solvent for synthesis an purification of pharmaceutical intermediates, a solvent for synthesis and purification of DNA, a solvent for synthesis of organic EL materials, and a cleaning solvent for electronic parts, and which is also purified to a level sufficiently usable as a mobile phase solvent for high-performance liquid chromatography.

### [Best Mode for Carrying Out the Invention]

The following will specifically describe the invention.

The invention lies on a process for producing highly pure acetonitrile, which comprises mixing hydrous crude acetonitrile with an alkali, separating the resulting mixture into an acetonitrile phase and an aqueous phase, removing the aqueous phase, passing the resulting acetonitrile phase through a distillation step to obtain purified acetonitrile, and passing the resulting purified acetonitrile though a cation-exchange resin to obtain highly pure acetonitrile.

The hydrous crude acetonitrile means acetonitrile prepared by recovering and purifying crude acetonitrile obtained as a by-product in the production of acrylonitrile or methacrylonitrile by catalytic ammoxydation of propylene, propane, isobutene, or isobutane and is acetonitrile containing 10 to 80% by weight of acetonitrile, 20 to 90% by weight of water, and other many kinds of impurities. With regard to the hydrous crude acetonitrile, allyl alcohol is preferably separated beforehand, for example, by the method described in the above Patent Document 8. Moreover, it is preferred that the hydrous crude acetonitrile is reacted with an aqueous sodium hydroxide solution or an aqueous potassium hydroxide solution beforehand at a temperature of 20 to 80°C, preferably 60 to 75°C for 1 to 15 hours, preferably 3 to 10 hours to convert acrylonitrile and hydrocyanic acid into succinonitrile and polymerized products such as a dimer and they are removed as far as possible.

Acetonitrile and water form an azeotropic mixture. Therefore, contrivance is necessary for lowering the content of water to azeotropic composition or less. As processes hitherto known, there may be mentioned a process of removing water by shifting the azeotropic composition with swinging distillation pressure from a low pressure to a high pressure (see, e.g., Patent Document 5), a process of extractive distillation with adding benzene (see, e.g., Patent Document 6), a process of extractive dehydration with adding an alkali (see, e.g., Patent Document 7), and a process of extractive dehydration with adding calcium chloride.

The process of the invention is characterized by extractive dehydration with adding an alkali. Namely, an acetonitrile phase is obtained by mixing hydrous crude acetonitrile with an alkali in an amount sufficient to extract water present therein and subsequently removing a separated aqueous layer. At that time, as the extracting agent for water, it is preferred to use an aqueous solution or a solid matter of sodium hydroxide and/or potassium hydroxide. Preferred is an aqueous solution.

The temperature for the extraction is from 5°C to 60°C, preferably from 10°C to 35°C.

The amount of the alkali to be used varies depending on the moisture contained in the crude acetonitrile but usually is in the range of 10 to 90% by weight, preferably 30 to 60% by weight relative to the moisture contained in the crude acetonitrile. By the process, the moisture contained in the acetonitrile is reduced to 10% by weight or less, particularly 3% by weight or less. The contact of the hydrous crude acetonitrile with the aqueous alkali solution is preferably a continuous counter-current contact. As a packing for a counter-current contact tower, Raschig ring, Lessing ring, Paul ring, Berle saddle, interlock saddle, Teralet packing, Dickson ring, and MacMahon packing are preferred and as a regular packing, a packing having a reticulate structure is preferred.

The distillation tower for use in the invention is a plate tower or a packed tower.

As examples of the plate tower, there may be mentioned a cross-current contact type with a down corner, or a counter-current contact type without a down comer. Moreover, as an opening of a tray, a bubble type, a porous plate type, or a valve type can be used. The number of plates of the distillation tower is not particularly limited as far as it is 10 plates or more but a tower having 30 to 80 plates is preferred.

As an example of the packed tower, a tower packed with an irregular packing and/or a regular packing as a packing can be used. As the irregular packing, for example, Raschig ring, Lessing ring, Paul ring, Berle saddle, interlock saddle, Teralet packing, Dickson ring, or MacMahon packing can be used. As the regular packing, a packing having a reticulate structure can be used. As materials for these irregular and regular packings, those made of porcelain, metal, plastic, or carbon can be employed. Moreover, the packed tower may be provided with a liquid-redistribution plate at an appropriate height to enhance efficiency of vapor-liquid contact.

In the invention, as a process of separating and removing compounds having a low boiling point and compounds having a high boiling point as compared with acetonitrile, a process using two distillation towers may be mentioned. Specifically, there may be mentioned a process of separating and removing the compounds having a low boiling point from the top in the first tower, separating and removing the compounds having a high boiling point from the bottom in the second tower, and obtaining purified acetonitrile from the top; or a process of separating and removing the compounds having a high boiling point from the bottom in the first tower, separating and removing the compounds having a low boiling point from the top in the second tower, and obtaining purified acetonitrile from the bottom or an upper part thereof. Moreover, as a modification thereof, they may be taken out as a side stream at an upper part of the bottom instead of the bottom or as a side stream of a lower part of the top instead of the top.

In these distillation steps, the reflux ratio and the discharged amounts of the compounds having a low boiling point and the compounds having a high boiling point can be determined as conditions for obtaining purified acetonitrile which matches the purpose. The discharged amount containing the compounds having a low boiling point from the top and the discharged amount containing the compounds having a high boiling point from the bottom relative to the acetonitrile fed are both 0.01% or more, preferably 0.1% or more.

Preferably, the distillation step comprises a step of refluxing main acetonitrile from the top of a distillation tower to the distillation tower via a condenser and discharging the remainder to outside of the distillation tower and a step of recovering purified acetonitrile from the bottom of the tower and a reflux ratio of the distillation tower is from 5 to 50. The ratio is more preferably from 10 to 30, most preferably from 15 to 25. The reflux ratio is defined as a value obtained by dividing the weight to be refluxed to the distillation tower by the weight to be discharged outside of the distillation tower. When the reflux ratio is lower than 5, the purity of the highly pure acetonitrile lowers and when the reflux ratio is larger than 50, energy efficiency decreases.

The lower limit of pressure of the distillation tower is 0.05 MPa or more, preferably 0.08 MPa or more, particularly preferably 0.09 MPa or more as absolute pressure and the upper limit is 0.27 MPa or less, preferably 0.20 MPa or less, particularly preferably 0.15 MPa or less as absolute pressure.

The distillation tower preferably has a bottom temperature of 80 to 95°C, particularly preferably 80 to 84°C and a top temperature of 70 to 80°C, particularly preferably 75 to 80°C.

Purified acetonitrile is obtained by the above steps and highly pure acetonitrile can be produced by passing the purified acetonitrile through a cation-exchange resin.

In the invention, as the cation-exchange resin, a porous-type or gel-type strongly acidic cation-exchange resin or weakly acidic cation-exchange resin can be employed. A sulfonic acid group can be exemplified as a strongly acidic group, and a carboxylic acid group as a weakly acidic group. Moreover, either type of resins of ion-exchange resins for aqueous solutions or ion-exchange resins for nonaqueous solutions can be used.

As the strongly acidic cation-exchange resin, for example, Amberlite (1006FH, IRP-69, IR-118, IRC-76, IR120BHAG, etc.) and Amberlyst (15H, 15DRY, 15JWET, 16WET, 31WET, 35WET, XN1010, etc.) manufactured by Rohm and Haas, Diaion (SK1B, SK104, SK110, SK112, SK116, PK208, PK212, PK216, PK220, PK22, UBK550, PK208, PK208, UBK530, etc.) manufactured by Mitsubishi Chemical Corporation, Dowex Monosphere (650C, 650HXC, 650HXCNG, 575CNG, 650CUPW, 650CNG, 575C, MP525C, 750C) and Dowex (HCR-NG, HCR-W2, HGRW2, MSC1C, 50, 88, 88MB, etc.) manufactured by Dow Chemical Company, and MP-62 manufactured by Bayer AG can be exemplified.

As the weakly acidic cation-exchange resin, for example, Amberlite IRP-64, Amberlite (IRC-76, FPC-3500, IRC-50S), and Diaion WK (10, 11, 100, 01S, 40, etc.) can be exemplified.

Preferably used is a strongly acidic ion-exchange resin for nonaqueous solutions, and Amberlite 1006FH, Amberlyst (15H, 15DRY, 15JWET, 16WET, 31WET, 35WET), Dowex Monosphere 650C, Dowex HCR-W2 can be exemplified.

The cation-exchange resin is used in a protonic form. In the case of a commercially available product which is not a protonic form or in the case that there is such a possibility, it is necessary to use the cation-exchange resin after the resin is treated with an acidic aqueous solution such as an aqueous nitric acid solution, an aqueous sulfuric acid solution, or an aqueous hydrochloric acid solution. It is preferred to use the resin after the above treated resin is washed by bringing it into contact with a sufficient amount of pure water and then washed by bringing it into contact with a sufficient amount of acetonitrile to remove the moisture and impurities contained in the ion-exchange resin.

It is preferred that the size of the cation-exchange resin is from 0.001 to 100 mm, further from 0.01 to 20 mm.

The contact of the cation-exchange resin with the purified acetonitrile can be conducted by a continuous method or a batch method but industrially, it is preferably conducted by a continuous method. As the continuous method, part of a packed tower or a pipe or the like is packed with the cation-exchange resin and the purified acetonitrile is fed, brought into contact therewith, and passed through. Moreover, the operation is preferably conducted at a space velocity SV of 0.001 to 1000 (l/min), preferably 0.01 to 10 (1/min), particularly preferably 0.04 to 1 (l/min). The temperature for the treatment with the cation-exchange resin is preferably from 5°C to 70°C, particularly preferably from 20°C to 60°C.

Important items of quality as highly pure acetonitrile are the content of propionitrile and the absorbance in ultraviolet absorption at a wavelength of 200 nm, and it is necessary that both satisfy the following requirements.
· The content of propionitrile is 150 ppm by weight or less, preferably 40 ppm by weight or less, further preferably 25 ppm by weight or less.
· The absorbance in ultraviolet absorption at a wavelength of 200 nm is 0.30 abs/cm or less, preferably 0.25 abs/cm or less, further preferably 0.20 abs/cm or less.

### [Examples]

The following will describe the invention with reference to Examples and Comparative Examples. The ultraviolet absorbance was measured by the following method.

Using an automatic spectrophotometer UV3101PC manufactured by Shimadzu Corporation, the absorbance at a wavelength range of 190 to 500 nm was measured using distilled water as a reference liquid with a quartz absorption cell of 1 cm square. The scanning rate is medium speed and the set value of slit width of the spectrophotometer is 5 nm.

### [Comparative Example 1]

Acetonitrile, a by-product of ammoxydation of propylene was concentrated by distillation, made pH 13 with sodium hydroxide, and reacted at 75°C for 6 hours, and substances having a high boiling point, such as allyl alcohol and succinonitrile, were separated by distillation. Then, after the water content was reduced to 5% by weight with sodium hydroxide, atmospheric distillation was conducted twice at a reflux ratio of 15 in a distillation tower having 50 plates to remove water, substances having a low boiling point, and substances having a high boiling point, whereby purified acetonitrile was obtained. When the purified acetonitrile was analyzed, the absorbance in ultraviolet absorption at a wavelength of 200 nm was 0.57 abs/cm and propionitrile was 20 ppm by weight.

### [Example 1]

A column having a diameter of 27 mm was packed with a cation-exchange resin (Amberlyst 15JWET) so that the layer height became 700 mm. The packed amount is 400 mL. At 20°C, 400 mL of the purified acetonitrile of Comparative Example 1 was passed through at a liquid-passing rate of 100 (mL/min). Upon analysis, the absorbance in ultraviolet absorption at a wavelength of 200 nm was 0.17 abs/cm, the absorbance in ultraviolet absorption at a wavelength of 254 nm was 0.001 abs/cm, and propionitrile was 20 ppm by weight.

### [Comparative Example 2]

Purified acetonitrile was obtained by repeating Example 1 except that an anion-exchange resin (Amberlyst A-21) was used. When the purified acetonitrile was analyzed, the absorbance in ultraviolet absorption at a wavelength of 200 nm was 2.0 abs/cm and propionitrile was 20 ppm by weight.

### [Comparative Example 3]

Acetonitrile, a by-product of ammoxydation of propylene was concentrated by distillation, made pH 13 with sodium hydroxide, and reacted at 75°C for 6 hours, and substances having a high boiling point, such as allyl alcohol and succinonitrile, were separated. Then, the distillation in Comparative Example 1 was repeated except that reduced-pressure distillation was conducted at 0.02 MPa as absolute pressure and subsequently pressure distillation was conducted at 0.25 MpaG as gauge pressure to remove water, substances having a low boiling point, and substances having a high boiling point, whereby purified acetonitrile was obtained. When the purified acetonitrile was analyzed, the absorbance in ultraviolet absorption at a wavelength of 200 nm was 0.62 abs/cm and propionitrile was 210 ppm by weight.

### [Comparative Example 4]

Purified acetonitrile was obtained by repeating the method of Example 1 except that the acetonitrile obtained in Comparative Example 3 was used. When the purified acetonitrile was analyzed, the absorbance in ultraviolet absorption at a wavelength of 200 nm was 0.45 abs/cm and propionitrile was 210 ppm by weight.

From the above Examples and Comparative Examples, it is realized that acetonitrile obtained by the process of the invention has a remarkably small content of propionitrile and also a small content of impurities which absorb ultraviolet ray at a wavelength of 200 nm.

### [Industrial Applicability]

The invention is a process which requires a small consumption of energy for use in purification and simple purification facility and steps. Therefore, it is suitable as a process for providing highly pure acetonitrile having a small absorbance in an ultraviolet absorption at a wavelength of 200nm, which has a small content of propionitrile for applications such as a solvent for synthesis an purification of pharmaceutical intermediates, a solvent for synthesis and purification of DNA, a solvent for synthesis of organic EL materials, and a cleaning solvent for electronic parts, and which is also purified to a level sufficiently usable as a mobile phase solvent for high-performance liquid chromatography with suppressing an increase in cost to a minimum level.

## Claims

1. A process for producing highly pure acetonitrile, which comprises mixing hydrous crude acetonitrile with an alkali, separating the resulting mixture into an acetonitrile phase and an aqueous phase, removing the aqueous phase to reduce the moisture contained in the acetonitrile to 10 % by weight or less, distilling the resulting acetonitrile phase from 0.05 MPa to 0.27 MPa of pressure of the distillation tower to obtain purified acetonitrile, and passing the resulting purified acetonitrile through a cation-exchange resin to obtain highly pure acetonitrile,
wherein the highly pure acetonitrile is highly pure acetonitrile having a content of propionitrile of 150 ppm by weight or less and an absorbance in ultraviolet absorption at 200 nm of 0.3 abs/cm or less.

2. The process for producing highly pure acetonitrile according to claim 1, wherein the distillation step comprises a step of refluxing from a top of a distillation tower to the distillation tower via a condenser and discharging the remainder to outside of the distillation tower and a step of recovering purified acetonitrile from a bottom of the tower, and a ratio of the reflux of the distillation tower is from 5 to 50.

3. The process for producing highly pure acetonitrile according to claim 1 or 2, wherein the alkali is sodium hydroxide and/or potassium hydroxide.

4. The process for producing highly pure acetonitrile according to any one of claims 1 to 3, wherein the acetonitrile phase is distilled to carry out removal of components having a boiling point lower than that of acetonitrile and removal of components having a boiling point higher than that of acetonitrile.

5. The process for producing highly pure acetonitrile according to any one of claims 1 to 4, wherein the cation-exchange resin is a strongly acidic cation-exchange resin.

## Patentansprüche

1. Verfahren zur Herstellung von hochreinem Acetonitril, umfassend das Mischen von wässrigem Roh-Acetonitril mit Alkali, Trennen des erhaltenen Gemischs in eine Acetonitrilphase und eine wässrige Phase, Entfernen der wässrigen Phase, sodass die in dem Acetonitril enthaltene Feuchtigkeit auf 10 Gew.-% oder weniger vermindert wird, Destillieren der erhaltenen Acetonitrilphase bei einem Destillationsturmdruck von 0,05 MPa bis 0,27 MPa, um gereinigtes Acetonitril zu erhalten, und Führen des erhaltenen gereinigten Acetonitrils durch ein Kationenaustauschharz, um hochreines Acetonitril zu erhalten,
wobei das hochreine Acetonitril ein hochreines Acetonitril mit einem Propionitrilgehalt von 150 Gew.-ppm oder weniger und einer Absorption bei Ultraviolettabsorption bei 200 nm von 0,3abs/cm oder weniger ist.

2. wVerfahren zur Herstellung von hochreinem Acetonitril nach Anspruch 1, wobei der Destillationsschritt einen Rückflussschritt von oberhalb des Destillationsturms zum Destillationsturm über einen Kondensator und die Abgabe des Rückstands zur Außenseite des Destillationsturms und einen Schritt des Rückgewinnens von gereinigtem Acetonitril vom Boden des Turms umfasst, wobei das Rückflussverhältnis des Destillationsturms von 5 bis 50 ist.

3. Verfahren zur Herstellung von hochreinem Acetonitril nach Anspruch 1 oder 2, wobei das Alkali Natriumhydroxid und/oder Kaliumhydroxid ist.

4. Verfahren zur Herstellung von hochreinem Acetonitril nach einem der Ansprüche 1 bis 3, wobei die Acetonitrilphase destilliert wird, um Komponenten mit einem niedrigeren Siedepunkt als dem von Acetonitril zu entfernen, und um Komponenten mit einem höheren Siedepunkt als dem von Acetonitril zu entfernen.

5. Verfahren zur Herstellung von hochreinem Acetonitril nach einem der Ansprüche 1 bis 4, wobei das Kationenaustauschharz ein stark saures Kationenaustauschharz ist.

## Revendications

1. Procédé de production d'acétonitrile hautement pur, lequel comprend le mélange d'acétonitrile brut hydraté avec un alcali, la séparation du mélange résultant en une phase d'acétonitrile et en une phase aqueuse, l'élimination de la phase aqueuse pour réduire l'humidité contenue dans l'acétonitrile à 10 % en poids ou inférieure, la distillation de la phase d'acétonitrile résultante de 0,05 MPa à 0,27 MPa de pression de la tour de distillation pour obtenir de l'acétonitrile purifié, et le passage de l'acétonitrile purifié résultant à travers une résine échangeuse de cations pour obtenir de l'acétonitrile hautement pur,
dans lequel l'acétonitrile hautement pur est de l'acétonitrile hautement pur ayant une teneur en propionitrile de 150 ppm en poids ou inférieure et une capacité d'absorption dans une absorption d'ultraviolet à 200 nm de 0,3 abs/cm ou inférieure.

2. Procédé de production d'acétonitrile hautement pur selon la revendication 1, dans lequel l'étape de distillation comprend une étape de reflux à partir d'une tête d'une tour de distillation vers la tour de distillation via un condenseur et de décharge du reste vers l'extérieur de la tour de distillation et une étape de récupération d'acétonitrile purifié à partir d'une queue de la tour, et un taux du reflux de la tour de distillation est de 5 à 50.

3. Procédé de production d'acétonitrile hautement pur selon la revendication 1 ou 2, dans lequel l'alcali est l'hydroxyde de sodium et/ou l'hydroxyde de potassium.

4. Procédé de production d'acétonitrile hautement pur selon l'une quelconque des revendications 1 à 3, dans lequel la phase d'acétonitrile est distillée pour réaliser l'élimination de constituants ayant un point d'ébullition inférieur à celui de l'acétonitrile et l'élimination de constituants ayant un point d'ébullition supérieur à celui de l'acétonitrile.

5. Procédé de production d'acétonitrile hautement pur selon l'une quelconque des revendications 1 à 4, dans lequel la résine échangeuse de cations est une résine échangeuse de cations fortement acide.
